# EUROPEAN PATENT APPLICATION

(11) **EP 4 609 837 A1**
(43) Date of publication of application: **03.09.2025**
(21) Application number: 23881890.0
(22) Date of filing: 25.10.2023
(51) Int. Cl.: A61F 2/966, A61F 2/24

(54) **CONVEYING DEVICE AND PROSTHESIS SYSTEM**

(30) Priority: 27.10.2022 CN 202222850460 U; 27.10.2022 CN 202211326255
(71) Applicant: Enlight Medical Technologies (Shanghai) Co., Ltd., Shanghai 201318 (CN)
(72) Inventor: HU, Zhanming, Shanghai 201315 (CN); ZHOU, Guanguan, Shanghai 201315 (CN); LI, Wensi, Shanghai 201315 (CN); LI, Fang, Shanghai 201315 (CN)
(74) Representative: Balder IP Law, S.L.
(86) International application number: PCT/CN2023/126554
(87) International publication number: WO 2024/088313

(57) **Abstract**

A conveying device (100), comprising: a pull rod assembly (110), a handle housing (120), and a control wire (130). The pull rod assembly (110) comprises an axis (123) and a pull rod (111), and the pull rod (111) comprises a middle portion (112) and first interlocking components (113) connected to the middle portion (112). The handle housing (120) comprises a handle casing (121) and second interlocking components (122) provided on the handle casing (121). The proximal end of the control wire (130) is connected to the middle portion (112). The first interlocking components (113) and the second interlocking components (122) are configured to be mutually locked under the action of elastic force; the elastic force can be overcome so that the first interlocking components (113) and the second interlocking components (122) are unlocked. The present application further provides a prosthesis system. According to the conveying device (100) and the prosthesis system provided by the present application, after a prosthesis is conveyed to a target position in a human body, the probability of sliding of the control wire (130) can be further reduced when the control wire (130) does not need to be operated, and then unnecessary movement of the prosthesis is further avoided.

## Description

### CROSS-REFERENCE TO RELATED APPLICATIONS

The present application is based on Chinese patent application No. 202211326255.8 filed on October 27, 2022 and Chinese patent application No. 202222850460.6 filed on October 27, 2022, and claims priority to these Chinese patent applications. The entire contents of these Chinese patent applications are hereby incorporated into the present application by reference.

### TECHNICAL FIELD

Embodiments of the present disclosure relate to the technical field of medical devices, and in particular to a delivery device and a prosthesis system.

### BACKGROUND

With the continuous development of medical technology, the concept of "minimal invasion" has been widely adopted across various fields of surgical treatment.

Currently, during the implementation of minimally invasive surgeries generally, a prosthesis is often delivered to a target position through surgical pathways such as human lumens by means of a delivery device. After reaching the target position, the prosthesis is then indirectly manipulated by controlling a control wire of the delivery device, thereby changing its state to achieve the surgical purpose. When manipulating the control wire of the delivery device, a delivery assembly connected to a proximal end of the control wire is generally driven manually, for example, by rotation or translation, so as to manipulate the control wire, thereby adjusting the state of the prosthesis connected to the distal end of the control wire.

When manipulation of the control wire is not required, in order to prevent unintended movement of the control wire caused by movement of the delivery assembly which may result in undesired movement of the prosthesis, a protrusion is generally provided on the delivery assembly, and multiple detent grooves are provided on the housing of the delivery device to engage with the protrusion. When the prosthesis moves to a target state, the delivery assembly is rotated to allow the protrusion to be threaded into one of the detent grooves, achieving relative fixation between the delivery assembly and the housing of the delivery device. However, with such a configuration, the protrusion is prone to slipping out of the detent groove, causing the delivery assembly to move relative to the housing of the delivery device, which may result in the failure of the entire prosthesis system.

### SUMMARY

Some embodiments of the present disclosure aim to provide a delivery device and a prosthesis system, where the delivery device is less prone to malfunction when manipulation of the control wire is not required, thereby preventing the prosthesis system from failing.

In order to address one or more of the above-mentioned problems, some embodiments of the present disclosure provide a delivery device including: a pull rod assembly, a handle housing and a control wire. The pull rod assembly includes an axis and a pull rod including a main body portion and a first interlocking component connected to the main body portion. The handle housing includes a housing body and a second interlocking component disposed on the housing body. A proximal end of the control wire is connected to the main body portion and a distal end of the control wire is configured to control the prosthesis to perform a first motion. The first interlocking component and the second interlocking component are configured to interlock each other under an action of elastic force, so that the main body portion and the housing body remain relatively fixed. The elastic force is able to be overcome to unlock the first interlocking component from the second interlocking component, allowing the pull rod to move relative to the handle housing.

Some embodiments of the present disclosure provide a prosthesis system, including: a prosthesis and the above-mentioned delivery device, where the distal end of the control wire is connected to the prosthesis.

The delivery device and prosthesis system provided in the embodiments of the present disclosure can deliver the prosthesis to the target position in the human body by the delivery device during minimally invasive surgeries, and then control the movement of the prosthesis by the control wire of the delivery device, thereby changing the state of the prosthesis to achieve the surgical purpose. When manipulation of the control wire is required, the first interlocking component and the second interlocking component are unlocked by overcoming the elastic force, so that the pull rod can move relative to the handle housing, enabling manipulation of the control wire to control the first motion of the prosthesis through the control wire connected to the prosthesis. When manipulation of the control wire is not required, the first interlocking component and the second interlocking component are locked by elastic force, so that the pull rod remains in a relatively fixed relationship with the handle housing to avoid the slippage of the control wire and the unintended state change of the prosthesis. Such configuration can effectively avoid the unintended movement of the pull rod for manipulating the control wire, which causes the failure of the prosthesis system.

### BRIEF DESCRIPTION OF THE DRAWINGS

One or more embodiments are described exemplarily with reference to the accompanying drawings, and these exemplary descriptions do not constitute a limitation on the embodiments. Elements having the same reference numeral in the accompanying drawings indicate similar elements. The figures in the accompanying drawings do not constitute a scale limitation unless otherwise stated.
Fig. 1 is a cross-sectional view of a delivery device provided in some embodiments of the present disclosure;
Fig. 2 is a partially enlarged view of portion A in Fig. 1;
Fig. 3 is a schematic structural diagram of a pull rod and a central rod provided in some embodiments of the present disclosure, viewed from a perspective perpendicular to that of Fig. 1;
Fig. 4 is a cross-sectional view of the interlocking portion between a pull rod assembly and a handle housing provided in some embodiments of the present disclosure;
Fig. 5 is a cross-sectional view of a partial structure of the handle housing provided in some embodiments of the present disclosure;
Fig. 6 is a schematic diagram of a mitral valve clip provided in some embodiments of the present disclosure;
Fig. 7 is a schematic structural diagram of an arm and an actuating assembly of the mitral valve clip, provided in some embodiments of the present disclosure;
Fig. 8 is a schematic structural diagram of a base, a backspan, and a locking assembly of the mitral valve clip, provided in some embodiments of the present disclosure;
Fig. 9 is a cross-sectional view of the base, the backspan, and the locking assembly of the mitral valve clip, provided in some embodiments of the present disclosure;
Fig. 10 is a schematic structural diagram of the backspan provided in some embodiments of the present disclosure; and
Fig. 11 is a schematic structural diagram of a lock plate provided in some embodiments of the present disclosure.

### DETAILED DESCRIPTION OF THE EMBODIMENTS

In order to make the objects, technical schemes and advantages of embodiments of the present disclosure clearer, the embodiments of the present disclosure will be described in detail below with reference to the accompanying drawings. However, those of ordinary skill in the art may understand that in the embodiments of the present disclosure, many technical details are set forth in order to make the reader better understand the present disclosure. However, the technical solutions set forth in the present disclosure may still be implemented even without these technical details and various changes and modifications based on the following embodiments.

**In** the embodiments of the present disclosure, terms such as "up", "down", "left", "right", "front", "rear", "top", "bottom", "inside", "outside", "middle", "vertical", "horizontal", "transverse", "longitudinal", etc., indicating orientations or positional relationships, are based on the orientations or positional relationships shown in the accompanying drawings. These terms are mainly intended to better describe the present disclosure and its embodiments, and are not intended to limit the indicated devices, elements, or components to have a specific orientation, or to be constructed and operated in a specific orientation.

Moreover, in addition to indicating orientations or positional relationships, the aforementioned terms may also have other meanings. For instance, the term "up" may also be used to indicate a certain attachment or connection relationship in certain situations. For those skilled in the art, the specific meanings of these terms in the present disclosure should be understood based on the specific circumstances.

In addition, the terms "installation", "arrangement", "provided with", "opened", "connection", and "connected" should be understood in a broad sense. For example, the "connection" may be a fixed connection, a detachable connection, or an integral structure; a mechanical connection or an electrical connection; may be a direct connection, an indirect connection through an intermediary, or internal communication between two devices, elements, or components. For those skilled in the art, the specific meanings of these terms in the present disclosure should be understood based on the specific circumstances.

In addition, the terms "first", "second", etc. are mainly used to distinguish different devices, elements, or components (which may or may not have the same specific type and structure), and are not intended to indicate or imply the relative importance and quantity of the indicated devices, elements, or components. The expression "multiple" or "a plurality of" indicates two or more unless otherwise specified.

In some embodiments of the present disclosure, "proximal end" or "proximal side" refers to the end closer to the operator and farther away from the patient. Correspondingly, "distal end" or "distal side" refers to the end closer to the patient and farther away from the operator. In some embodiments of the present disclosure, the terms "parallel" and "vertical" should not be narrowly interpreted as 0° or 90°, but should be understood as reasonable angle ranges including 0° or 90° with some permissible fluctuation, provided that the technical effects are achieved.

Some embodiments of the present disclosure provide a delivery device, including: a pull rod assembly, a handle housing and a control wire. The pull rod assembly includes an axis and a pull rod including a main body portion and a first interlocking component connected to the main body portion. The handle housing includes a housing body and a second interlocking component disposed on the housing body. A proximal end of the control wire is connected to the main body portion, and a distal end of the control wire is configured to control the prosthesis to perform a first motion. The first interlocking component and the second interlocking component are configured to interlock each other under an action of elastic force, so that the main body portion and the housing body remain relatively fixed. The elastic force is able to be overcome to unlock the first interlocking component from the second interlocking component, allowing the pull rod to move relative to the handle housing.

The delivery device provided in some embodiments of the present disclosure can deliver the prosthesis to the target position in the human body by the delivery device during minimally invasive surgeries, and then control the movement of the prosthesis by the control wire of the delivery device, thereby changing the state of the prosthesis to achieve the surgical purpose. When manipulation of the control wire is required, the first interlocking component and the second interlocking component are unlocked by overcoming the elastic force, so that the pull rod can move relative to the handle housing, enabling manipulation of the control wire to control the first motion of the prosthesis through the control wire connected to the prosthesis. When manipulation of the control wire is not required, the first interlocking component and the second interlocking component are locked by elastic force, so that the pull rod remains in a relatively fixed relationship with the handle housing to avoid the slippage of the control wire and the unintended state change of the prosthesis. Such configuration can effectively avoid the unintended movement of the pull rod for manipulating the control wire, which causes the failure of the prosthesis system.

When the prosthesis is driven to perform the first motion by means of the control wire, the state of the prosthesis can be changed. The "change of state" here may refer to the changes in the structure, size, and shape of the entire or part of prosthesis. For example, the prosthesis changes from a compressed state to an expanded state, such as a covered stent used for treating aortic stenosis. A restraint wire is typically used to restrain the covered stent in a compressed state during delivery. Once the covered stent is positioned at the target location, the restraint wire is withdrawn from the covered stent by the control wire, allowing the covered stent to transition from a compressed state to an expanded state. The "change of state" may also refer to the implementation of the unlocked state/locked state between the prosthesis and the target tissue, between the prosthesis and the delivery system, and between the internal components of the prosthesis, and/or the mutual transformation between the locked state and the unlocked state. For example, when delivering a mitral valve clip, the locking assembly is connected to the control wire, and the arm is locked by the locking assembly. When the mitral valve clip needs to be released, the control wire is driven towards the proximal end to unlock the locking assembly, which in turn unlocks the arm and allows it to rotate. The "change of state" may also refer to changes in the position and/or posture of the entire or part of the prosthesis. For example, in the case of the mitral valve clip used for treating mitral valve regurgitation, the control wire is used for controlling the relative rotation of the arm of the mitral valve clip, thereby achieving the clamping of the mitral valve leaflets.

In order to make the objects, technical schemes and advantages of the present disclosure clearer, the embodiments of the present disclosure will be described in detail below with reference to the accompanying drawings.

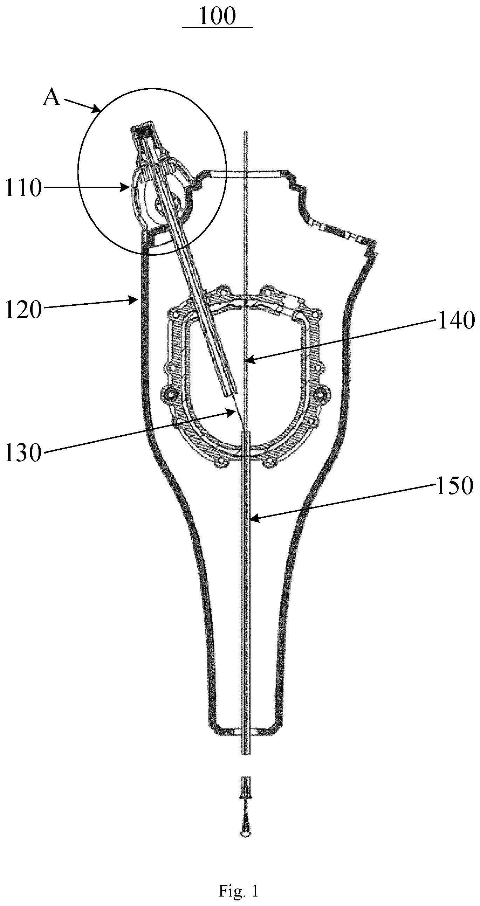

Referring to Figs. 1 to 5, some embodiments of the present disclosure provide a delivery device 100 including a pull rod assembly 110, a handle housing 120 and a control wire 130. The pull rod assembly 110 includes an axis 123 and a pull rod 111 including a main body portion 112 and a first interlocking component 113 connected to the main body portion 112. The handle housing 120 includes a housing body 121 and a second interlocking component 122 disposed on the housing body 121. A proximal end of the control wire 130 is connected to the main body portion 112, and a distal end of the control wire 130 is connectable to a prosthesis and configured to control the prosthesis to perform a first motion. The first interlocking component 113 and the second interlocking component 122 are configured to interlock each other under an action of elastic force, so that the main body portion 112 and the housing body 121 remain relatively fixed. The elastic force is able to be overcome to unlock the first interlocking component 113 from the second interlocking component 122, allowing the pull rod 111 to move relative to the handle housing 120.

In this way, when manipulation of the control wire 130 is not required, the first interlocking component 113 and the second interlocking component 122 can be locked by the action of elastic force, thereby ensuring that the pull rod 111 and the handle housing 120 remain relatively fixed, avoiding undesired movement of the prosthesis connected to the distal end of the control wire 130 caused by unintended movement of the pull rod 111, which changes the state of the prosthesis.

In some embodiments, the pull rod 111 further includes an elastic portion 114 for providing an elastic force acting on the first interlocking component 113. Thus, when manipulation of the control wire 130 is not required, the elastic portion 114 provides the elastic force acting on the first interlocking component 113, thereby achieving the interlocking between the first interlocking component 113 and the second interlocking component 122. When manipulation of the control wire 130 is required, the elastic force provided by the elastic portion 114 and acting on the first interlocking component 113 is overcome, thereby achieving the unlocking between the first interlocking component 113 and the second interlocking component 122. For example, the elastic portion 114 is an elongated elastic member as shown below.

It should be noted that the present disclosure does not limit the positional relationship between the first interlocking component 113 and the second interlocking component 122.

In some embodiments, the handle housing 120 may be provided with an elastic portion for providing an elastic force acting on the second interlocking component 122. In this case, when manipulation of the control wire 130 is not required, the elastic portion of the handle housing 120 provides the elastic force acting on the second interlocking component 122, thereby achieving the interlocking between the second interlocking component 122 and the first interlocking component 113. When manipulation of the control wire 130 is required, the elastic force provided by the elastic portion of the handle housing 120 and acting on the second interlocking component 122 is overcome, thereby achieving the unlocking between the first interlocking component 113 and the second interlocking component 122. For example, the elastic portion is a spring. For example, the elastic portion is a pressure spring. The second interlocking component 122 is movably disposed on the housing body 121 and interlocks with the first interlocking component 113 under the action of the pressure spring.

In some embodiments, the pull rod 111 may include an elastic portion 114, and the handle housing 120 may include another elastic portion. The elastic portion 114 of the pull rod 111 is configured to provide an elastic force acting on the first interlocking component 113, and the elastic portion of the handle housing 120 is configured to provide an elastic force acting on the second interlocking component 122. When manipulation of the control wire 130 is not required, the elastic portion 114 of the pull rod 111 provides an elastic force acting on the first interlocking component 113, and the elastic portion of the handle housing 120 provides an elastic force acting on the second interlocking component 122, thereby achieving the interlocking between the first interlocking component 113 and the second interlocking component 122. When manipulation of the control wire 130 is required, the elastic force provided by the elastic portion 114 of the pull rod 111 and acting on the first interlocking component 113 is overcome, and the elastic force provided by the elastic portion of the handle housing 120 and acting on the second interlocking component 122 is also overcome, thereby unlocking the first interlocking component 113 and the second interlocking component 122. For example, the elastic portion 114 of the pull rod 111 is an elongated elastic member as shown below; the elastic portion of the handle housing 120 is a pressure spring; and the second interlocking component 122 is movably disposed on the housing body 121. Under the respective actions of the elongated elastic member and the pressure spring, the first interlocking component 113 and the second interlocking component 122 interlock with each other.

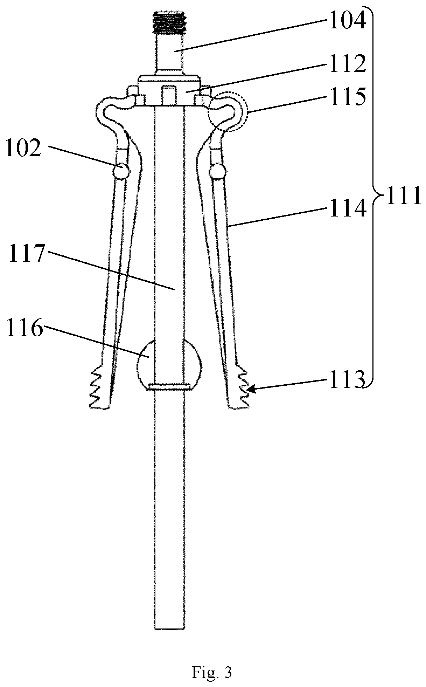

Referring to Figs. 3 to 5, in the case that the pull rod 111 includes an elastic portion 114, the first interlocking component 113 is arranged between the axis 123 of the pull rod assembly 110 and the second interlocking component 122; and the elastic force provided by the elastic portion 114 drives the first interlocking component 113 to interlock with the second interlocking component 122. In this way, the elastic portion 114 can exert an elastic force on the first interlocking component 113 in a direction away from the axis 123 of the pull rod assembly 110, so that the first interlocking component 113 and the second interlocking component 122 interlock with each other. And, the elastic force exerted by the elastic portion 114 can facilitate the interlocking between the first interlocking component 113 and the second interlocking component 122.

In some embodiments, when the elastic portion 114 is in a natural state without deformation, the distance between the second interlocking component 122 and the axis 123 of the pull rod assembly 110 is smaller than the distance between the first interlocking component 113 and the axis 123 of the pull rod assembly 110. When the first interlocking component 113 is engaged with the second interlocking component 122, the elastic portion 114 undergoes elastic deformation, enabling the first interlocking component 113 to interlock with the second interlocking component 122, thereby achieving a constant locked state, which is the default state.

In this manner, the elastic force provided by the elastic deformation of the elastic portion 114 serves as a preload force. During scenarios in which no operation of the control wire 130 is required by the operator-such as after the delivery device 100 has been assembled or during the delivery of the prosthesis to the vicinity of the target site-the first interlocking component 113 interlocks with the second interlocking component 122 and remains in a constant locked state. Such configuration can prevent inadvertent disengagement between the first interlocking component 113 and the second interlocking component 122 during the transportation of the delivery device 100, or degradation of the locking engagement due to repeated operations of the pull rod assembly 110 during the surgical process. The failure of the locking may result in unintended displacement of the pull rod assembly 110 and compromise the reliability of the prosthesis system.

Referring to Fig. 3, the elastic portion 114 is elongated in shape, and its proximal end is connected to the main body portion 112. The first interlocking component 113 is disposed at the distal end of the elastic portion 114. And the distance between the distal end of the elastic portion 114 and the axis 123 of the pull rod assembly 110 is greater than the distance between the proximal end of the elastic portion 114 and the axis 123 of the pull rod assembly 110. The elastic portion 114 may be made of elastic material. For example, the elastic portion 114 is made of metal material (such as stainless steel, titanium alloy) or the elastic portion 114 is made of elastic polymer material (such as polycarbonate). Thus, this configuration facilitates the arrangement in which the distance between the second interlocking component 122 and the axis 123 of the pull rod assembly 110 is smaller than the distance between the first interlocking component 113 and the axis 123 of the pull rod assembly 110; and it also allows that the elastic portion 114 undergoes elastic deformation to generate an elastic force acting on the first interlocking component 113. In one example, the distance between the elastic portion 114 and the axis 123 of the pull rod assembly 110 gradually increases in a direction directing from the proximal end of the pull rod assembly 110 towards the distal end.

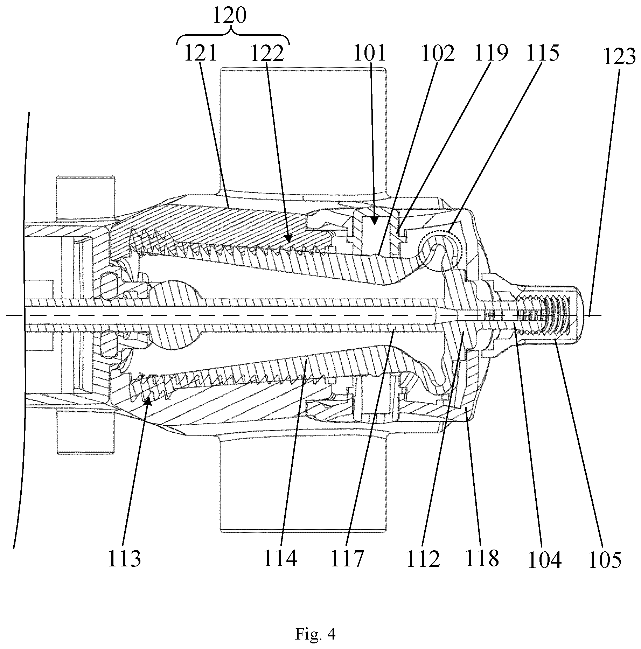

In some embodiments, as shown in Figs. 3 and 4, the elastic portion 114 has a bending portion 115 at the proximal end for enhancing elastic force. The elastic portion 114 is connected to the main body portion 112 at the bending portion 115. This configuration allows the elastic force generated by the deformation of the elastic portion 114 to be adjusted by the bending portion 115, thereby balancing the stability and the tactile feedback during the unlocking operation after the first interlocking component 113 and the second interlocking component 122 interlock with each other.

It should be noted that the specific form of the bending portion 115 is not limited in the present disclosure. It may be in, for example, a "U" shape, "V" shape, "S" shape, "Z" shape, etc. In one example, the bending portion 115 is U-shaped, that is, the bending portion 115 extends from the proximal end of the elastic portion 114 towards or away from the axis 123 of the pull rod assembly 110, and then reversely extends to connect with the main body portion 112.

In an embodiment, the elastic portion 114 generates elastic force by the bending portion 115, while the remaining portion of the elastic portion 114 or even the entire elastic portion 114 may be made of a material that is not easily deformed. In this way, the selection range of materials for preparing the elastic portion 114 is increased.

In some embodiments, the pull rod assembly 110 further includes a central rod 117 with a hollow structure. The central rod 117 is configured to constrain the extension direction of the control wire 130. The proximal end of the central rod 117 is connected to the main body portion 112, and the distal end of the central rod 117 is a free end accommodated in the handle housing 120. The proximal end of the control wire 130 is fixed to the main body portion 112. The control wire 130 enters the central rod 117 from its proximal end, exits the central rod 117 from its distal end to enter the handle housing 120, and then exits the handle housing 120. The control wire 130 accommodated inside the central rod 117 is constrained by the central rod 117, so that the extension direction of the part of the control wire 130 is same as the extension direction of the central rod 117. In an embodiment, the axis of the central rod 117 may coincide with the axis 123 of the pull rod assembly 110.

In some embodiments, the pull rod 111 further includes a limiting portion 116 arranged on the axis 123 of the pull rod assembly 110. The limiting portion 116 is configured to prevent the elastic portion 114 from undergoing plastic deformation when the elastic portion 114 deforms to generate elastic force. In this way, when the first interlocking component 113 moves away from the second interlocking component 122 to achieve an unlocking state between the first interlocking component 113 and the second interlocking component 122, that is, when the elastic force exerted by the elastic portion 114 is overcome, the limiting portion 116 is used for preventing the first interlocking component 113 from moving excessively, thereby causing plastic deformation of the elastic portion 114, and ensuring the reliability of the delivery device 100. As shown in Fig. 3 and Fig. 4, the axis of the central rod 117 may coincide with the axis 123 of the pull rod assembly 110, and the limiting portion 116 is disposed on the central rod 117. During the process in which the elastic portion 114 is driven to deflect toward the axis 123 of the pull rod assembly 110 to achieve unlocking of the first interlocking component 113 and the second interlocking component 122, the elastic portion 114 abuts against the limiting portion 116 when it deflects excessively, preventing the limiting portion 116 from further deflecting and avoiding plastic deformation of the elastic portion 114. There are no specific limitation on the specific position of the limiting portion 116 along the axis 123 of the pull rod assembly 110 and the size of the limiting portion 116, which can be selected based on the material properties of the elastic portion 114. For example, when the material of the elastic portion 114 allows significant elastic deformation, the limiting portion 116 is far away from the main body portion 112 and the size of the limiting portion 116 is smaller; and when the material of the elastic portion 114 allows only minor elastic deformation, the limiting portion 116 is closer to the main body portion 112 and the size of the limiting portion 116 is larger. In some embodiments, the shape of the limiting portion 116 may be spherical, cylindrical, conical, drum-shaped, etc. In one example, the limiting portion 116 is also configured to be disposed on the distal end of the central rod 117 for abutting against the handle housing 120 to prevent the pull rod assembly 110 from excessively advancing into the handle housing 120.

Further referring to Figs. 1 to 5, in some embodiments, the pull rod assembly 110 further includes a pull rod housing 118 and an unlocking component 119. The pull rod housing 118 is fixed to the main body portion 112, and the unlocking component 119 is movably disposed on the pull rod housing 118 for exerting external force on the elastic portion 114 to overcome the elastic force. Such configuration allows the operator to use the unlocking component 119 to achieve unlocking between the first interlocking component 113 and the second interlocking component 122 in a comfortable manner.

It should be noted that the present disclosure does not limit the specific implementation of using the unlocking component 119 to achieve unlocking between the first interlocking component 113 and the second interlocking component 122. For example, the first interlocking component 113 is connected to the main body portion 112 via the elastic portion 114, and the elastic portion 114 is arranged between the axis 123 of the pull rod assembly 110 and the unlocking component 119 at least in a direction perpendicular to the axis 123 of the pull rod assembly 110. The unlocking component 119 is configured to be movable relative to the pull rod housing 118 in a direction perpendicular to the axis 123 of the pull rod assembly 110. When the unlocking component 119 moves towards the axis 123 of the pull rod assembly 110, it exerts a force on the elastic portion 114, so that the elastic portion 114 moves towards the axis 123 of the pull rod assembly 110, causing the first interlocking component 113 to move relative to the second interlocking component 122 towards the axis 123 of the pull rod assembly 110, thus achieving unlocking. When the external force exerted on the unlocking component 119 is removed, the elastic portion 114 recovers its original state under its inherent elasticity and drives the unlocking component 119 to move away from the axis 123 of the pull rod assembly 110.

Referring to Fig. 4, the elastic portion 114 is arranged between the axis 123 of the pull rod assembly 110 and the unlocking component 119. The proximal end of the elastic portion 114 (that is, the end fixed to the main body portion 112) is closer to the axis 123 of the pull rod assembly 110 than the distal end of the elastic portion 114 (that is, the end where the first interlocking component 113 is disposed). When the unlocking component 119 moves relative to the pull rod housing 118 in a direction perpendicular to the axis 123 of the pull rod assembly 110 and toward the axis 123, the elastic portion 114 deflects towards the axis 123 of the pull rod assembly 110.

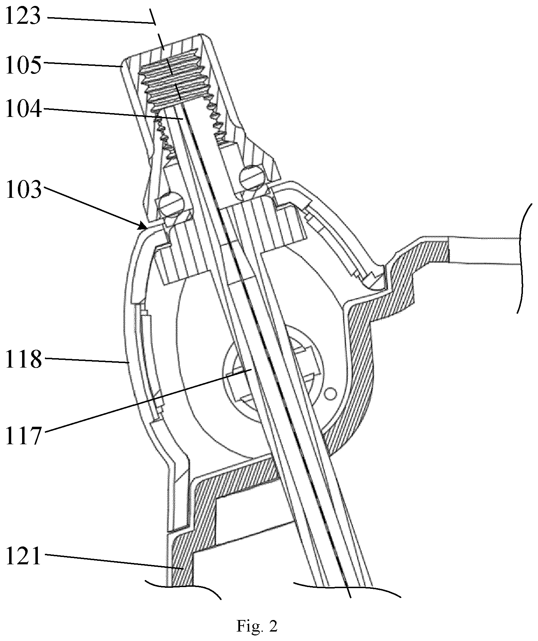

Further referring to Figs. 2 to 4, the unlocking component 119 is provided with a first engaging portion 101 at a side facing the elastic portion 114, and the elastic portion 114 is provided with a second engaging portion 102 at a side facing the unlocking component 119. The first engaging portion 101 is configured to engage with the second engaging portion 102. Such configuration can prevent the unlocking component 119 from moving relative to the elastic portion 114.

In some embodiments, the first engaging portion 101 may be a groove recessed away from the elastic portion 114 from the side of the unlocking component 119 facing the elastic portion 114, and the second engaging portion 102 may be a protrusion protruding towards the unlocking component 119 from the side of the elastic portion 114 facing the unlocking component 119. The protrusion extends into the groove. In some embodiments, the first engaging portion 101 may be a protrusion protruding towards the elastic portion 114 from the side of the unlocking component 119 facing the elastic portion 114, and the second engaging portion 102 may be a groove recessed away from the unlocking component 119 from the side of the elastic portion 114 facing the unlocking component 119. The protrusion extends into the groove. In some embodiments, the groove and the protrusion also extend in a direction perpendicular to the axis 123 of the pull rod assembly 110 and perpendicular to the direction of movement of the unlocking component 119 (that is, a direction perpendicular to the viewing plane of Fig. 4), to increase the contact area between the two.

Further referring to Figs. 2, 4, and 5, the pull rod housing 118 includes a proximal surface 103 provided with an opening. The main body portion 112 is arranged on the distal side of the proximal surface 103, that is, inside the pull rod housing 118. The pull rod 111 further includes a proximal extension 104 and a fixing member 105. The distal end of the proximal extension 104 is connected to the main body portion 112 and extends outward from the proximal surface 103 to form a free end. The fixing member 105 cooperates with the free end of the proximal extension 104, so that the main body portion 112 and the fixing member 105 respectively engage with two sides of the proximal surface 103, thereby achieving the fixation between the pull rod 111 and the pull rod housing 118.

It should be noted that the present disclosure does not limit the specific implementation of the cooperation between the fixing member 105 and the free end of the proximal extension 104. In one example, the fixing member 105 is fixed to the proximal extension 104 via a snap-fit, and abuts against the proximal side of the proximal surface 103. In another example, the fixing member 105 is fixed to the proximal extension 104 by interference fit, and abuts against the proximal side of the proximal surface 103. In yet another example, the fixing member 105 is fixed to the proximal extension 104 by threaded connection, and abuts against the proximal side of the proximal surface 103.The free end of the proximal extension 104 may be provided with external threads, and the fixing member 105 may be provided with internal threads that mate with the external threads of the proximal extension 104.

In addition, when the fixing member 105 is engaged with the free end of the proximal extension 104, the proximal end of the control wire 130 can also be fixed by the engagement between the fixing member 105 and the proximal extension 104. In one example where the free end of the proximal extension 104 is provided with external threads and the fixing member 105 is provided with internal threads that mate with the external threads of the proximal extension 104, the proximal end of the control wire 130 is wrapped around the external threads of the proximal extension 104, and then is fixed by the engagement between the internal threads of the fixing member 105 and the external threads of the proximal extension 104.

In some embodiments, there are multiple first interlocking components 113, which are evenly arranged around the axis 123 of the pull rod assembly 110; the number of the second interlocking components 122 is equal to that of the first interlocking components 113, and the second interlocking components 122 are arranged in one-to-one correspondence with the first interlocking components 113. In this way, the interlocking between the multiple first interlocking components 113 and the corresponding second interlocking components 122 improves the stability of the locked connection between the pull rod assembly 110 and the handle housing 120. Correspondingly, in some embodiments, there may also be provided multiple elastic portions 114 and multiple unlocking components 119 as described in the aforementioned content. Both the elastic portions 114 and the unlocking components 119 are arranged in one-to-one correspondence with the first interlocking components 113.

Referring to Figs. 3 and 4, the number of first interlocking components 113 is two. The interlocking between the two first interlocking components 113 and the corresponding second interlocking components 122 improves the stability of the locked connection between the pull rod assembly 110 and the handle housing 120, while also avoiding an excessive number of first interlocking members 113, which may otherwise hinder manual operation of the operator.

In some embodiments, the number of the first interlocking components 113 may also be one, three, or four, which is not limited here.

In some embodiments, the first interlocking component 113 is provided with at least one first locking portion, and the second interlocking component 122 is provided with multiple second locking portions. The number of the second locking portions is greater than that of the first locking portions. Multiple first locking portions are arranged sequentially along a first predetermined direction, and multiple second locking portions are arranged sequentially along a second predetermined direction. Each of the first locking portions can selectively lock with one of the multiple second locking portions. The second predetermined direction is parallel to the axis 123 of the pull rod assembly 110, or, an angle between the second predetermined direction and the axis 123 of the pull rod assembly 110 is less than an angle between the first predetermined direction and the axis 123 of the pull rod assembly 110. In one example, the first predetermined direction may be the axis extension direction of the elastic portion 114; and the second predetermined direction is the extension direction of the inner wall of the housing body 121. The number of the second locking portions is determined according to the stroke of the pull rod 111. Multiple first locking portions help to improve the stability of locking between the first interlocking component 113 and the second interlocking component 122 after locking.

After the operator exerts an external force to overcome the elastic force to unlock the first interlocking component 113 and the second interlocking component 122, the pull rod assembly 110 may be pushed or pulled along its axis 123, thereby driving the control wire 130 to achieve indirectly manipulation of the control wire 130. When the pull rod assembly 110 is pushed or pulled along the axial direction of the pull rod assembly 110, since the second predetermined direction is parallel to the axis 123 of the pull rod assembly 110; or, the angle between the second predetermined direction and the axis 123 of the pull rod assembly 110 is less than the angle between the first predetermined direction and the axis 123 of the pull rod assembly 110, and since the multiple second locking portions are arranged sequentially along the second predetermined direction, the first locking portion can still lock with one of the multiple second locking portions after displacement. Accordingly, the first interlocking component 113 can lock with the second interlocking component 122 again, thereby maintaining the moved pull rod assembly 110 fixed relative to the handle housing 120. In the case that the second predetermined direction is parallel to the axis 123 of the pull rod assembly 110, the external force exerted required to overcome the elastic force during moving the pull rod assembly 110 is uniform, and the push-pull process is smooth. In the case that the angle between the second predetermined direction and the axis 123 of the pull rod assembly 110 is less than the angle between the first predetermined direction and the axis 123 of the pull rod assembly 110, greater external force is required to overcome the elastic force as the pull rod assembly 110 is moved towards the proximal end, so that the operator can get a clear feeling that the pull rod assembly 110 is approaching the proximal limit position.

In some embodiments, when the first interlocking component 113 is positioned at the distal end of the second interlocking component 122, the angle between the first predetermined direction and the second predetermined direction ranges from 6° to 9°.

In some embodiments, the first locking portion is a first toothed structure with the tooth tip facing the second locking portion; the second locking portion is a second toothed structure with the tooth tip facing the first locking portion; the first toothed structure and the second toothed structure are configured to mesh with each other to achieve interlocking of the first locking portion and the second locking portion.

In some embodiments, the number of first toothed structures is 3 to 6; and the number of the second toothed structures is 15 to 20.

In some embodiments, the first toothed structure and the second toothed structure are obtuse or right triangles in shape, with the tips of the triangles inclined towards the distal end of the delivery device 100. Thus, it is more effort-saving when the pull rod assembly 110 moves towards the proximal end.

It should be noted that the delivery device 100 provided in the present disclosure can be widely applied and can be used in any field that requires to achieve change of state of the prosthesis through movement of the control wire 130.

For example, the prosthesis may be a covered stent used for treating aortic stenosis. A restraint wire is typically used during delivery to restrain the covered stent in a compressed state. After the covered stent is positioned at the target position, the restraint wire is released by the control wire 130, allowing the covered stent to transition from a compressed state to an expanded state. The prosthesis may also be a valve clip used for repairing the valve to treat valve regurgitation. The control wire 130 can control the rotation of the arm in the valve clip, or achieve locking and unlocking of the arm in the valve clip.

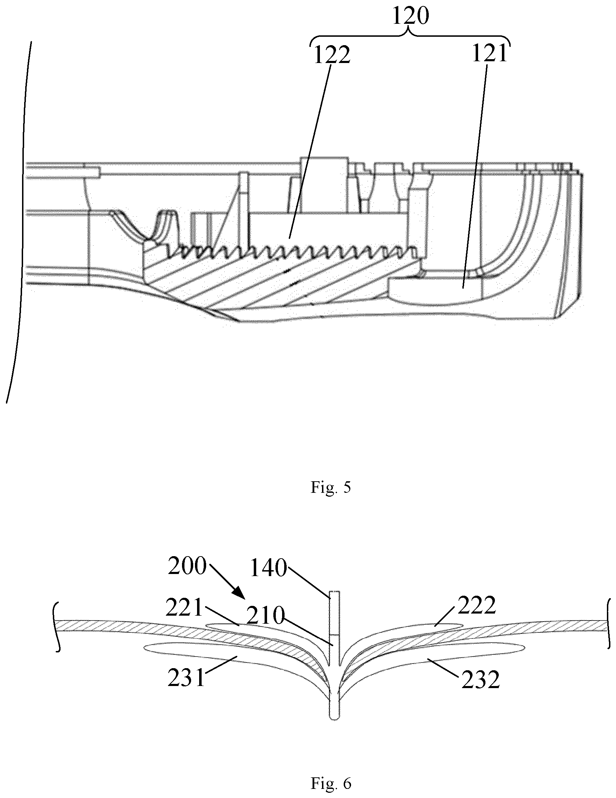

Referring to Fig. 6, a prosthesis, specifically a valve clip for treating mitral regurgitation, namely the mitral valve clip 200, is taken as an example for further explanation. It should be noted that the prosthesis here is a mitral valve clip 200, which is only an example. In other embodiments, the prosthesis may also be a tricuspid valve clip or other valve clip for treating other valve regurgitation, or a prosthesis for treating other diseases.

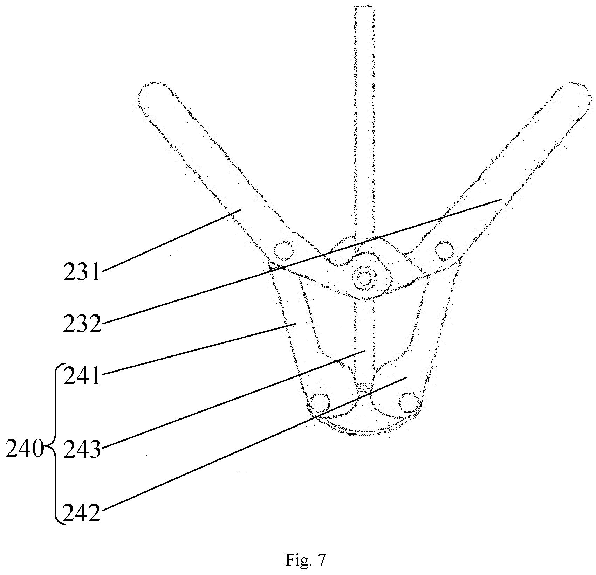

As shown in Fig. 6 and Fig. 7, the mitral valve clip 200 includes a base 210, an arm, and a gripper. The arm is disposed at the distal end of the gripper, and a clamping space is formed between the gripper and the arm to accommodate the mitral valve leaflet. The arm and the gripper can rotate around the axis of the base 210 to move close to or away from the base 210, respectively. Correspondingly, the clamping space can be enlarged to facilitate the entry of the leaflet, and the clamping space can be reduced to clamp the leaflet. Thus, the relative motion between the arm and the gripper achieves the clamping of the mitral valve leaflet. In some embodiments, there are two arms and two grippers, which are symmetrically arranged at two ends of the base 210.

For example, the grippers include a first gripper 221 and a second gripper 222, and the arms include a first arm 231 and a second arm 232. The first gripper 221 and the second gripper 222 are symmetrically arranged about the axis of the base 210 and arranged in the plane defined by the first arm 231 and the second arm 232, to better clamp the edges of the anterior leaflet and posterior leaflet of the mitral valve. Here, the plane defined by the first arm231 and the second arm 232 refers to the plane formed by the axis of the first arm 231 and the axis of the second arm 232. The first gripper 221 and the second gripper 222 being arranged in the plane defined by the first arm 231 and the second arm 232 refers to that the axes of the first gripper 221 and the second gripper 222 are both located in the plane defined by the first arm 231 and the second arm 232. In one embodiment, the first gripper 221 and the second gripper 222 can rotate independently. In one embodiment, the first arm 231 and the second arm 232 are rotatably connected to the base 210 at different pivot joints, respectively. In one embodiment, the first arm 231 and the second arm 232 are rotatably connected to the backspan 243, via a same rotating shaft.

Referring to Figs. 7, 8 and 10, the mitral valve clip further includes an actuating assembly 240, which includes a first arm link 241, a second arm link 242, and a backspan 243. One end of the first arm link 241 is rotatably connected to the first arm 231, and the other end is rotatably connected to the backspan 243. One end of the second arm link 242 is rotatably connected to the second arm 232, and the other end is rotatably connected to the backspan 243. The backspan 243 is T-shaped, including a horizontal section 2432 and a vertical section 2431. Two ends of the horizontal section 2432 are rotatably connected to the first arm link 241 and the second arm link 242, respectively. An end of the vertical section 2431 is accommodated in the hollow base 210 and is movably connected to the base 210. Thus, the first arm link 241, the first arm 231, the backspan 243, and the base 210 form a first kinematic mechanism similar to a crank-slider mechanism. The second arm link 242, the second arm 232, the backspan 243, and the base 210 form a second kinematic mechanism similar to a crank-slider mechanism.

When a force is exerted on the backspan 243 by the delivery device 100, the backspan 243 moves relative to the base 210. As a result, the first arm 231 of the first kinematic mechanism is caused to rotate, whereby the first arm 231 cooperates with the first gripper 221 to reduce the clamping space, allowing one leaflet edge to be clamped. And the second arm 232 of the second kinematic mechanism is caused to rotate, whereby the second arm 232 cooperates with the second gripper 222 to reduce the clamping space, allowing the other leaflet edge to be clamped. In one example, the first arm link 241 is rotatably connected to the first arm 231 and the backspan 243 by means of pin connections; and the second arm link 242 is rotatably connected to the second arm 232 and the backspan 243 also by means of pin connections.

In some embodiments, the mitral valve clip 200 further includes a locking assembly 250 for preventing the backspan 243 from moving relative to the base 210, thereby achieving locking or unlocking of the arms.

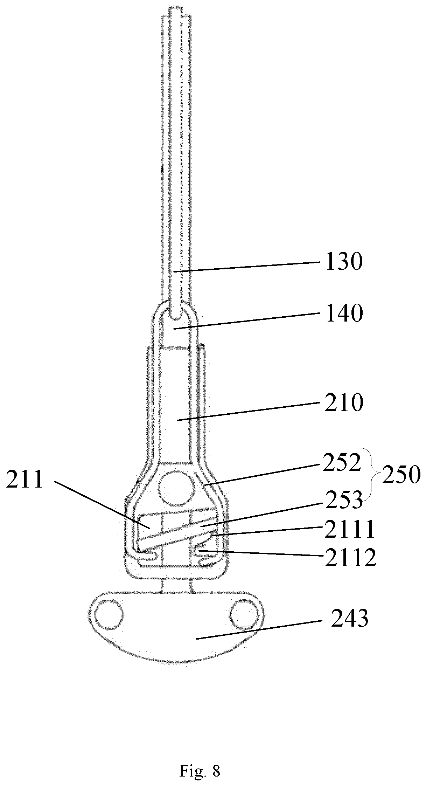

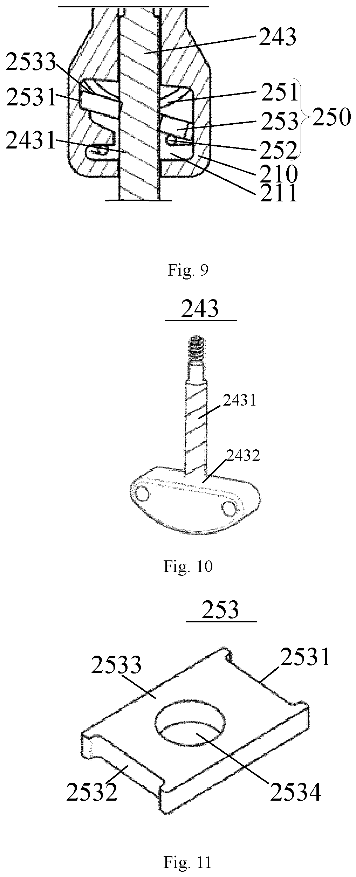

Referring to Figs. 8 and 9, the locking assembly 250 includes a clevis spring 251, a lock-release clip 252, and a lock plate 253. The distal end of the base 210 is provided with a locking slot 211 for accommodating the clevis spring 251 and the lock plate 253. The clevis spring 251 is configured to exert a locking force on the lock plate 253, so that the lock plate 253 can engage with the vertical section 2431 of the backspan 243 to prevent the actuating assembly 240 from moving, thereby achieving locking of the arms. The lock-release clip 252 is configured to exert an unlocking force on the lock plate 253 to overcome the locking force of the clevis spring 251, so that the lock plate 253 can be disengaged from the vertical section 2431 of the backspan 243, and the backspan 243 can move relative to the base 210, thereby achieving unlocking of the arms.

Referring to Fig. 11, the base 210 is provided with an axially extending accommodating channel. The lock plate 253 includes a first side edge 2531, a second side edge 2532, a first surface 2533, and an internal cavity 2534. The first side edge 2531 and the second side edge 2532 are arranged opposite to each other. The vertical section 2431 of the backspan 243 is accommodated in the accommodating channel and passes through the inner cavity 2534 of the lock plate 253. The first side edge 2531 of the lock plate 253 abuts against the locking slot 211, and the second side edge 2532 is connected to the lock-release clip 252. The first surface 2533 of the lock plate 253 abuts against the clevis spring 251. At this time, the arms are in a locked state.

In some embodiments, the clevis spring 251 is a leaf spring. Under the action of the clevis spring 251, the lock plate 253 is arranged in an inclined manner, such that the inner cavity 2534 of the lock plate 253 engages with the vertical section 2431 of the backspan 243. Furthermore, the edge between the side wall of the inner cavity 2534 of the lock plate 253 and the surface of the lock plate 253 is a sharp edge, that is, the edge is not chamfered. Furthermore, the surface of the vertical section 2431 of the backspan 210 is at least partially roughened to increase the friction between it and the inner cavity 2534 of the lock plate 253. Such configuration can improve the stability of the engagement between the lock plate 253 and the backspan 243.

In some embodiments, referring to Fig. 8, the lock-release clip 252 includes a first end, a second end, and a middle portion arranged between the first end and the second end. The first end extends within the locking slot 211 (in a direction perpendicular to the viewing plane of Fig. 8) and is secured within the locking slot 211. The middle portion is disposed on a lateral side of the base 210, extending towards the proximal end of the delivery device 100, then turning back and extending towards the distal end, forming a "U" shape. The second end also extends within the locking slot 211 (in a direction perpendicular to the viewing plane of Fig. 8) and is connected to the second side edge 2532 of the lock plate 253. The middle portion may be connected to the distal end of the control wire 130 to facilitate control of the control wire 130. When unlocking is needed, the unlocking component 119 is pressed to unlock the first interlocking component 113 and the second interlocking component 122 from the locked state, and then the pull rod 111 is driven to move towards the proximal end (that is, the first motion described above), thereby driving the control wire 130 to move towards the proximal end. The second end of the lock-release clip 252 then provides force to the second side edge 2532 of the lock plate 253, causing the lock plate 253 to overcome the force of the clevis spring 251 and rotate about a pivot point located at the abutment of the first side edge 2531 with the locking slot 211. At this time, the inner cavity of the lock plate 253 no longer contacts with the vertical section 2431 of the backspan 243. And the arms are in an unlocked state. When the arms need to be locked, the unlocking component 119 can be released to restore the first interlocking component 113 and the second interlocking component 122 to the locked state, so that the lock plate 253 maintains its original state. In this way, the vertical section 2431 of the backspan 243 can freely move in the axial direction, and the arms can be manipulated to freely rotate. The connection between the middle portion of the lock-release clip 252 and the control wire 130 may be implemented by bundling, bonding, or interpenetrating, or other means.

In some embodiments, the locking slot 211 includes a support platform 2111 protruding towards the inner wall of the locking slot 211. The first side edge 2531 of the lock plate 253 abuts against the support platform 2111. The locking slot 211 further includes a fixed platform 2112 protruding towards the inner wall of the locking slot 211. The fixed platform 2112 is disposed at the distal end of the support platform 2111 and is configured to retain the first end of the lock-release clip 252.

Further referring to Figs. 1 to 11, the delivery device may further include: a main transmission assembly 140. The main transmission assembly 140 includes a drive shaft assembly and a driving wire disposed coaxially with the drive shaft assembly. The drive shaft assembly is connected to the proximal end of the driving wire. The driving wire is detachably connectable to the prosthesis and configured to drive the prosthesis to perform a second motion.

In some embodiments, the vertical section 2431 of the backspan 243 is detachably connected to the distal end of the driving wire after passing through the inner cavity of the lock plate 253 via, for example, a threaded connection. The lock-release clip 252 is driven to move by the control wire 130, thereby exerting an unlocking force on the lock plate 253 to overcome the locking force of the clevis spring 251, so that the lock plate 253 is disengaged from the vertical section 2431 of the backspan 243, thereby achieving unlocking of the arms. Then, the driving wire is driven to move by the drive shaft assembly, which in turn drives the backspan 243 to move, causing the first arm 231 and the second arm 232 to rotate (that is, the second motion described above). The drive shaft assembly is configured to convert rotational movement into axial movement. For example, the drive shaft assembly includes a drive shaft and a shaft sleeve that is threadedly connected to the drive shaft, with the shaft sleeve remaining stationary relative to the housing body. Therefore, the drive shaft moves axially while the shaft sleeve receives external force to rotate.

As shown in Fig. 1, the delivery device 100 may further include an inner tube 150 disposed within the handle housing 120 along the axial direction of the delivery device 100. The main transmission assembly 140 is disposed coaxially with the inner tube 150, and the axis 123 of the pull rod assembly 110 intersects with the axis of the inner tube 150. As such, the driving wire extends from the drive shaft through the inner tube 150 and extends distally from the inner tube 150 to the mitral valve clip 200. The control wire 130 extends smoothly from the distal end of the pull rod assembly 110 into the inner tube 150, and extends distally from the inner tube 150 to the mitral valve clip 200, connecting with the lock-release clip 252 of the locking assembly 250.

In some embodiments, the delivery device 100 may also be provided with other wires (not shown in the figure), with a proximal end of the wire being disposed on the proximal end of the delivery device 100 and a distal end of the wire being connected to the gripper of the mitral valve clip 200. In the case that the gripper includes a first gripper 221 and a second gripper 222, the number of wires may be two, with the distal end of one of the wires being connected to the first arm 221 and the distal end of the other wire connected to the second arm 222. The first gripper 221 and the second gripper 222 are controlled respectively by the two wires to rotate.

It should be noted that in some embodiments, even if the prosthesis is a mitral valve clip 200, the control wire 130 may be used for controlling other components of the mitral valve clip 200 to perform the first motion, in addition to controlling the locking assembly 250 to lock the backspan 243 as described in the previous embodiment. For example, the distal end of the control wire 130 is connected to the gripper of the mitral valve clip 200 to control the rotation of the gripper(that is, the first motion described above). In the case that the grippers include a first arm 221 and a second arm 222, one control wire 130 may be used for driving the two grippers to rotate, or alternatively, two pull rod assemblies and two control wires 130 may be provided to respectively control the rotation of the first arm 221 and the rotation of the second arm 222, to change the state of the grippers. In addition to controlling part of the prosthesis to perform the first motion, the control wire 130 may also be used for controlling the entire prosthesis to perform the first motion in some embodiments, in order to change the overall state.

Referring to Figs. 1 to 11, some embodiments of the present disclosure provide a prosthesis system including a prosthesis and the delivery device as described in any one of the previous embodiments. The distal end of the control wire 130 is configured to control the prosthesis to perform the first motion. The prosthesis may be the prosthesis described in the previous embodiments (such as the mitral valve clip 200), and the delivery device has the similar structure as the delivery device 100 described in the previous embodiments. Therefore, the prosthesis system provided in the embodiments of the present disclosure has the similar beneficial effects as the delivery device 100 described in the previous embodiments, and will not be further described here.

The prosthesis system provided in the embodiments of the present disclosure has been introduced in detail above. Specific examples have been illustrated in this paper to elaborate on the principles and embodiments of the present disclosure. The descriptions of the above embodiments are only intended to help understand the present disclosure. There may be changes in specific embodiments and application scope. In summary, the content of this specification should not be construed as a limitation on the present disclosure.

## Claims

1. A delivery device (100), comprising: a pull rod assembly (110), a handle housing (120) and a control wire (130);
wherein the pull rod assembly (110) includes an axis (123) and a pull rod (111) including a main body portion (112) and a first interlocking component (113) connected to the main body portion (112);
the handle housing (120) includes a housing body (121) and a second interlocking component (122) disposed on the housing body (121);
a proximal end of the control wire (130) is connected to the main body portion (112), and a distal end of the control wire (130) is configured to control a prosthesis to perform a first motion; and
the first interlocking component (113) and the second interlocking component (122) are configured to interlock each other under an action of elastic force, thereby keeping the main body portion (112) relatively fixed with respect to the handle body (121); wherein the elastic force is able to be overcome to unlock the first interlocking component (113) and the second interlocking component (122), thereby allowing the pull rod (111) to move relative to the housing body (121).

2. The delivery device (100) according to claim 1, wherein
the pull rod (111) further includes an elastic portion (114), the elastic portion (114) being configured to provide an elastic force acting on the first interlocking component (113);
or, the handle housing (120) further includes an elastic portion, the elastic portion being configured to provide an elastic force acting on the second interlocking component (122);
or, the pull rod (111) and the handle housing (120) each include an elastic portion, and the elastic portion (114) of the pull rod (111) being configured to provide an elastic force acting on the first interlocking component (113), and the elastic portion of the handle housing (120) being configured to provide an elastic force acting on the second interlocking component (122).

3. The delivery device (100) according to claim 2, wherein
the pull rod (111) includes the elastic portion (114);
the first interlocking component (113) is arranged between the axis (123) of the pull rod assembly (110) and the second interlocking component (122); and
the elastic force provided by the elastic portion (114) drives the first interlocking component (113) to interlock with the second interlocking component (122).

4. The delivery device (100) according to claim 3, wherein
when the elastic portion (114) is in a natural state without deformation, a distance between the second interlocking component (122) and the axis (123) of the pull rod assembly (110) is smaller than a distance between the first interlocking component (113) and the axis (123) of the pull rod assembly (110), so that when the first interlocking component (113) and the second interlocking component (122) are engaged, the first interlocking component (113) interlocks with the second interlocking component (122), thereby achieving a constant locked state.

5. The delivery device (100) according to any one claims 2 to 4, wherein
a proximal end of the elastic portion (114) is connected to the main body portion (112), the first interlocking component (113) is disposed at a distal end of the elastic portion (114), and a distance between the distal end of the elastic portion (114) and the axis (123) of the pull rod assembly (110) is greater than a distance between the proximal end of the elastic portion (114) and the axis (123) of the pull rod assembly (110).

6. The delivery device (100) according to claim 5, wherein
the elastic portion (114) has a bending portion (115) at the proximal end thereof, and the elastic portion (114) is connected to the main body portion (112) at the bending portion (115).

7. The delivery device (100) according to claim 3, wherein
the pull rod (111) further includes a limiting portion (116) disposed on the axis (123) of the pull rod assembly (110), and the limiting portion (116) is configured to prevent the elastic portion (114) from undergoing plastic deformation when the elastic force is generated by deformation of the elastic portion (114).

8. The delivery device (100) according to claim 3, wherein
the pull rod assembly (110) further includes a central rod (117) with a hollow structure, the central rod (117) is configured to constrain an extension direction of the control wire (130) passing therethrough, a proximal end of the central rod (117) is connected to the main body portion (112), and a distal end of the central rod (117) is a free end accommodated in the handle housing (120).

9. The delivery device (100) according to claim 2, wherein
the pull rod assembly (110) further includes a pull rod housing (118) and an unlocking component (119), wherein the pull rod housing (118) is fixed to the main body portion (112), and the unlocking component (119) is movably disposed on the pull rod housing (118) for exerting an external force on the elastic portion (114) to overcome the elastic force.

10. The delivery device (100) according to claim 9, wherein
the first interlocking component (113) is connected to the main body portion (112) via the elastic portion (114), and the elastic portion (114) is arranged between the axis (123) of the pull rod assembly (110) and the unlocking component (119) at least in a direction perpendicular to the axis (123) of the pull rod assembly (110);
the unlocking component (119) is configured to be movable relative to the pull rod housing (118) in a direction perpendicular to the axis (123) of the pull rod assembly, wherein when the unlocking component (119) moves towards the axis (123) of the pull rod assembly (110), it exerts a force on the elastic portion (114), so that the elastic portion (114) moves towards the axis (123) of the pull rod assembly (110), thereby causing the first interlocking component (113) to move relative to the second interlocking component (122) towards the axis (123) of the pull rod assembly (110).

11. The delivery device (100) according to claim 9, wherein
the unlocking component (119) is provided with a first engaging portion (101) at a side facing the elastic portion (114), and the elastic portion (114) is provided with a second engaging portion (102) at a side facing the unlocking component (119), wherein the first engaging portion (101) is configured to engage with the second engaging portion (102) to prevent the unlocking component (119) from moving relative to the elastic portion (114).

12. The delivery device (100) according to claim 9, wherein
the pull rod housing (118) includes a proximal surface (103) with an opening;
the main body portion (112) is disposed on a distal side of the proximal surface (103);
the pull rod (111) further includes a proximal extension (104) and a fixing member (105), wherein a distal end of the proximal extension (104) is connected to the main body portion (112) and extends outward from the opening of the proximal surface (103) to form a free end, the fixing member (105) is configured to cooperate with the free end of the proximal extension (104), so that the main body portion (112) and the fixing member (105) respectively abut against opposite sides of the proximal surface (103), thereby achieving fixation between the pull rod (111) and the pull rod housing (118).

13. The delivery device (100) according to claim 1, wherein
a plurality of the first interlocking components (113) are provided, and the plurality of the first interlocking components (113) are evenly arranged around the axis (123) of the pull rod assembly (110);
the number of the second interlocking components (122) is equal to that of the first interlocking components (113), and the second interlocking components (122) are arranged in one-to-one correspondence with the first interlocking components (113).

14. The delivery device (100) according to claim 1, wherein
the first interlocking component (113) is provided with at least one first locking portion, and the second interlocking component (122) is provided with a plurality of second locking portions, wherein the number of the second locking portions is greater than that of the first locking portions;
the plurality of the first locking portions are arranged sequentially along a first predetermined direction, and the plurality of the second locking portions are arranged sequentially along a second predetermined direction, wherein each of the plurality of the first locking portions is selectively lockable with one of the plurality of the second locking portions;
wherein the second predetermined direction is parallel to the axis (123) of the pull rod assembly (110); or, an angle between the second predetermined direction and the axis (123) of the pull rod assembly (110) is smaller than an angle between the first predetermined direction and the axis (123) of the pull rod assembly (110).

15. The delivery device (100) according to claim 14, wherein
when the first interlocking component (113) is positioned at the distal end of the second interlocking component (122), the angle between the first predetermined direction and the second predetermined direction ranges from 6° to 9°.

16. The delivery device (100) according to claim 14, wherein
the first locking portion is a first toothed structure with a tooth tip facing the second locking portion;
the second locking portion is a second toothed structure with a tooth tip facing the first locking portion; and
the first toothed structure and the second toothed structure mesh with each other to achieve locking of the first locking portion and the second locking portion.

17. The delivery device (100) according to claim 16, wherein
the number of the first toothed structure is 3 to 6; and
the number of the second toothed structure is 15 to 20.

18. The delivery device (100) according to claim 16, wherein
the first toothed structure and the second toothed structure are obtuse or right triangles, with the tips of the triangles inclined towards the distal end of the delivery device (100).

19. The delivery device (100) according to claim 1, further comprising:
a main transmission assembly (140);wherein the main transmission assembly (140) includes a drive shaft assembly and a driving wire disposed coaxially with the drive shaft assembly, wherein a distal end of the drive shaft assembly is connected to a proximal end of the driving wire, the driving wire is detachably connectable to the prosthesis and configured to drive the prosthesis to perform a second motion.

20. A prosthesis system, comprising: a prosthesis and the delivery device (100) according to any one of claims 1 to 19, wherein the distal end of the control wire (130) is configured to control the prosthesis to perform the first motion.
